(19)

Europäisches Patentamt

European Patent Office

Office européen des brevets

(11) **EP 4 321 558 A1**

(12) **EUROPEAN PATENT APPLICATION**
published in accordance with Art. 153(4) EPC

(43) Date of publication:
**14.02.2024 Bulletin 2024/07**

(21) Application number: **22804480.6**

(22) Date of filing: **14.04.2022**

(51) International Patent Classification (IPC):
*C08J 3/12* (2006.01)          *C08L 101/12* (2006.01)
*A61F 13/53* (2006.01)         *B01J 20/26* (2006.01)
*B01J 20/28* (2006.01)         *B01J 20/32* (2006.01)
*A61L 15/22* (2006.01)         *A61L 15/24* (2006.01)
*A61L 15/42* (2006.01)         *A61L 15/60* (2006.01)

(52) Cooperative Patent Classification (CPC):
**A61F 13/53; A61L 15/22; A61L 15/24; A61L 15/42;
A61L 15/60; B01J 20/26; B01J 20/28; B01J 20/32;
C08J 3/12; C08L 101/12**

(86) International application number:
**PCT/JP2022/017872**

(87) International publication number:
**WO 2022/244566 (24.11.2022 Gazette 2022/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.05.2021   JP 2021083788
20.12.2021   JP 2021205790**

(71) Applicant: **Sumitomo Seika Chemicals Co., Ltd.
Kako-gun, Hyogo 675-0145 (JP)**

(72) Inventors:
• **SAWAKI Hiroki
  Himeji-shi, Hyogo 672-8076 (JP)**
• **YAMAMOTO Tomoka
  Himeji-shi, Hyogo 672-8076 (JP)**

(74) Representative: **Vossius & Partner
Patentanwälte Rechtsanwälte mbB
Siebertstrasse 3
81675 München (DE)**

(54) **COATED RESIN PARTICLES AND METHOD FOR PRODUCING COATED RESIN PARTICLES**

(57)     The present invention is related to a coated resin particle that includes a water-absorbent resin particle, and a coating layer covering at least a part of a surface of the water-absorbent resin particle, in which the coating layer contains a compound A, and a compound B more water-soluble than the compound A.

EP 4 321 558 A1

**Description**

**Technical Field**

The present invention relates to a coated resin particle and a method for producing a coated resin particle.

**Background Art**

Water-absorbent resin particles are widely used in various fields of sanitary materials such as disposable diapers, hygiene products, and portable toilets; agricultural and horticultural materials such as water retention agents and soil improvement agents; and industrial materials such as waterproofing agents and condensation prevention agents. It is desirable that an absorber containing the water-absorbent resin particles has a better absorption rate of humor and the like to be absorbed. Regarding the absorber containing the water-absorbent resin particles, for example, it is disclosed in Patent Literature 1 that an absorber containing water-absorbing resin powder includes a layer, at least a part of the layer having a diffusible region where a material for improving diffusibility with a liquid permeation speed under load of 15 seconds or less is disposed.

**Citation List**

**Patent Literature**

[Patent Literature 1] Japanese Unexamined Patent Publication No. 2014-46020

**Summary of Invention**

**Technical Problem**

Water-absorbent resin particles used in an absorber core of a disposable diaper include two types of water-absorbent resin particles having a low water retention ability and water-absorbent resin particles having a high water retention ability. When the water-absorbent resin particles having a low water retention ability are used for the absorber core, the liquid is quickly permeated, but an amount of the liquid that can be retained after liquid absorption is poor. Therefore, sufficient dryness may not be obtained. In a case where the dryness is insufficient, a so-called re-wet, in which a sheet shaped absorber in a curved state absorbs the liquid, and thereafter, the absorbed liquid returns from the absorber to the wearer side of the diaper, may occur. When the water-absorbent resin particles having a high water retention ability are used, a larger amount of the liquid can be absorbed and retained, thereby maintaining a high dryness, whereas a permeation rate of the liquid may be lowered. As described above, since the permeation rate of the liquid into the absorber core is related to the dryness (re-wet amount) with a trade-off relationship, it is difficult to improve the permeation rate while suppressing re-wet after liquid absorption.

An object of the present invention is to provide a coated resin particle that can improve a permeation rate of a liquid while sufficiently suppressing an increase in an re-wet amount after liquid absorption for the practical purpose as compared to a case of using a conventional water-absorbent resin particle alone, by using the conventional water-absorbent resin particle in combination when the coated resin particle is used in an absorbent article, and a method for producing the same.

**Solution to Problem**

In an aspect of the present invention, provided is a coated resin particle that includes a water-absorbent resin particle, and a coating layer covering at least a part of a surface of the water-absorbent resin particle, in which the coating layer contains a compound A, and a compound B more water-soluble than the compound A.

In another aspect of the present invention, provided is a method for producing a coated resin particle that includes coating at least a part of a water-absorbent resin particle with a coating material to form a coating layer on at least a part of a surface of the water-absorbent resin particle, in which the coating material contains a compound A in a solution state or an emulsion state, and a compound B more water-soluble than the compound A.

**Advantageous Effects of Invention**

According to the present invention, it is possible to provide a coated resin particle that can improve a permeation rate of a liquid by using conventional water-absorbent resin particles in combination when the coated resin particles are used in an absorbent article while sufficiently suppressing an increase of an re-wet amount after liquid absorption for the practical purpose as compared to a case of using the conventional water-absorbent resin particles alone, and a method for producing the same.

**Brief Description of Drawings**

Fig. 1 is a schematic cross-sectional view showing an embodiment of a coated resin particle.
Fig. 2 is a schematic cross-sectional view showing an embodiment of an absorbent article.
Fig. 3 is a photograph showing an analysis result of a coated resin particle of Example 5 by Raman spectrometric method.
Fig. 4 is a photograph showing an analysis result of a coated resin particle of Example 12 by Raman spectrometric method.

**Description of Embodiments**

Hereinafter, some embodiments of the present invention will be described in detail. However, the present invention is not limited to the following embodiments.

In the present specification, "acrylic" and "methacrylic" are collectively referred to as "(meth)acrylic". "Acrylate" and "methacrylate" are also referred to as "(meth)acrylate". In a numerical value range described in a stepwise manner in the present specification, an upper limit value or a lower limit value of a numerical value range in a certain step can be optionally combined with an upper limit value or a lower limit value of a numerical value range in another step. In a numerical value range described in the present specification, the upper limit value or the lower limit value of the numerical value range may be replaced with the value shown in the examples. For materials exemplified in the present specification, one kind may be used alone, or two or more kinds may be used in combination. In a case where there are a plurality of substances corresponding to each of components in a composition, a content of each of the components in the composition means the total amount of the plurality of substances present in the composition unless otherwise specified.

[Coated Resin Particle]

A coated resin particle of the present embodiment includes a water-absorbent resin particle, and a coating layer covering at least a part of a surface of the water-absorbent resin particle.

The coating layer is preferably chemically and/or physically bonded to the surface of the water-absorbent resin particle so that the coating layer is not easily falloff from the water-absorbent resin particle in a state before the water-absorbent resin particle absorbs water. For example, fine recesses existing on the surface of the water-absorbent resin particle are filled with the coating layer to generate an anchor effect, thereby achieving the physical bond.

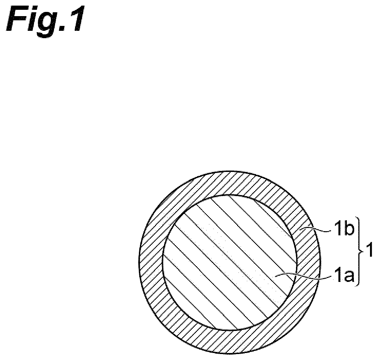

Fig. 1 is a schematic cross-sectional view showing an embodiment of the coated resin particle. As shown in Fig. 1, a coated resin particle 1 includes a water-absorbent resin particle 1a and a coating layer 1b with which at least a part of the surface of the water-absorbent resin particle 1a is coated. In Fig. 1, the entire surface of the water-absorbent resin particle 1 is coated with the coating layer 1b. The coating layer 1b contains a compound A and a compound B more water-soluble than the compound A.

The coated resin particle 1 is suppressed from absorbing water for a predetermined time after the coated resin particle 1 comes into contact with the liquid containing water because the coating layer 1b blocks contacting of the coated resin particle 1 with the liquid. When the coating layer 1b comes into contact with the liquid containing water, the compound B is gradually dissolved, and a portion in which the compound B was present corresponds to a water channel. Accordingly, the water-absorbent resin particle 1a absorbs the liquid intruded through the water channel and swells, while water absorption is suppressed in a portion where the compound A is present. It is considered that using the water-absorbent resin particle 1a of which the water absorption is suppressed as an absorber causes a phenomenon such that gaps existing between the water-absorbent resin particles are filled with swollen gel-like water-absorbent resin particles and the liquid hardly passes through the gaps (which is so-called gel blocking phenomenon) to be less likely to occur, thereby improving the permeation rate. In addition, it is considered that rewet after liquid absorption is also suppressed because the water-absorbent resin particle 1a gradually absorbs water via the water channel formed by the dissolution of the compound B even though absorption is suppressed. However, the mechanism by which the effect of the present invention is obtained is not limited to the above.

(Water-absorbent Resin Particle)

The water-absorbent resin particle may contain a polymer particle. The polymer particle may be a crosslinked polymer formed by polymerization of monomers including an ethylenically unsaturated monomer. The polymer particle can have a monomer unit derived from the ethylenically unsaturated monomer. The polymer particle can be produced by a method including a step of polymerizing monomers including ethylenically unsaturated monomers, for example. Examples of methods of polymerization include a reverse phase suspension polymerization method, an aqueous solution polymerization method, a bulk polymerization method, and a precipitation polymerization method.

The ethylenically unsaturated monomer may be a water-soluble ethylenically unsaturated monomer (an ethylenically unsaturated monomer having a solubility of 1.0 g or more in 100 g of water at 25°C). Examples of the water-soluble ethylenically unsaturated monomer include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where the ethylenically unsaturated monomer has an amino group, the amino group may be quaternized. The ethylenically unsaturated monomer may be used alone, or two or more kinds thereof may be used in combination.

In a case where the ethylenically unsaturated monomer has an acid group, the ethylenically unsaturated monomer may be used in the polymerization reaction after neutralizing the acid group with an alkaline neutralizing agent. The degree of neutralization of the ethylenically unsaturated monomer by the alkaline neutralizing agent may be 10% to 100% by mol, 50% to 90% by mol, or 60% to 80% by mol of the acid group in the ethylenically unsaturated monomer, for example.

From the viewpoint of industrial availability, the ethylenically unsaturated monomer may include at least one compound selected from the group consisting of (meth)acrylic acid and a salt thereof, acrylamide, methacrylamide, and N,N-dimethyl acrylamide. The ethylenically unsaturated monomer may include at least one compound selected from the group consisting of (meth)acrylic acid and a salt thereof, and acrylamide.

As a monomer for obtaining the water-absorbent resin particle, a monomer other than the above-mentioned ethylenically unsaturated monomer may be used. Such a monomer can be mixed and used with an aqueous solution containing the above-mentioned ethylenically unsaturated monomer, for example. The use amount of the ethylenically unsaturated monomer may be 70% to 100% by mol with respect to the total amount of monomers. The ratio of (meth)acrylic acid and a salt thereof may be 70% to 100% by mol with respect to the total amount of the monomers.

Crosslinking by self-crosslinking occurs during polymerization, but the crosslinking may be induced by using an internal crosslinking agent. When the internal crosslinking agent is used, water-absorbent characteristics (water retention capacity and the like) of the water-absorbent resin particle are easily controlled. The internal crosslinking agent is usually added to a reaction solution during the polymerization reaction.

A polymer on at least a surface layer portion of the polymer particle may be crosslinked by a reaction with a surface crosslinking agent. The surface crosslinking agent may be a compound containing two or more functional groups (reactive functional groups) having reactivity with a functional group derived from the ethylenically unsaturated monomer, for example.

The polymer particle may contain a certain amount of water, and may further contain various additional components therein, in addition to a polymer composed of the ethylenically unsaturated monomers. Examples of the additional components include a gel stabilizing agent and a metal chelating agent.

The shape of the water-absorbent resin particle is not particularly limited, and for example, the shape may be substantially spherical, crushed, or granular, or may be a shape that is formed of primary particles with these shapes aggregated.

The particle size distribution of the water-absorbent resin particles may be adjusted by performing an operation such as particle size adjustment using classification with a sieve, as necessary. For example, a fraction that passed through a sieve having an opening of 850 $\mu$m but did not pass through a sieve having an opening of 250 $\mu$m may be used as a water-absorbent resin particle.

(Coating Layer)

The coating layer contains a compound A and a compound B more water-soluble than the compound A. The compound A is a compound that is more hardly water-soluble than the compound B. A dissolved content of the compound A in 100 g of water at 25°C may be 1 % by mass or less. The dissolved content of the compound A in 100 g of water at 25°C may be, for example, 0.5% by mass or less, 0.3% by mass or less, or 0.1% by mass or less, or may be 0% by mass. The dissolved content of the compound A is measured by a method described in Examples described later.

The compound A may be a homopolymer or copolymer containing a substituted or unsubstituted alkene as a constituent unit (monomer unit). The copolymer containing the unsubstituted alkene as a constituent unit may be a copolymer containing only two or more unsubstituted alkenes as constituent units, may be a copolymer containing one or two or more unsubstituted alkenes and a monomer component other than an unsubstituted alkene as constituent units, or may be a copolymer containing one unsubstituted alkene and a monomer component other than an unsubstituted alkene as constituent units.

Examples of the unsubstituted alkene used in the copolymer include ethylene, propylene, and butene. The unsubstituted alkene used in the copolymer may be ethylene and/or propylene, or may be ethylene.

The substituted alkene used in the copolymer can be a halogenated alkene and/or an alkene substituted with a substituted or unsubstituted aromatic substituent (for example, a phenyl group or a naphthyl group). The substituted alkene used in the copolymer may be a halogenated alkene, may be halogenated vinyl (for example, chloroethylene, bromoethylene, or fluoroethylene) and/or ethylene substituted with a substituted or unsubstituted phenyl group, may be chloroethylene (vinyl chloride) and/or ethylene substituted with an unsubstituted phenyl group (styrene), or may be vinyl chloride.

As the monomer component other than the substituted or unsubstituted alkene used in the copolymer, a water-soluble ethylenically unsaturated monomer may be used. The compounds listed above can be used as the water-soluble ethylenically unsaturated monomer. The water-soluble ethylenically unsaturated monomer may be (meth)acrylic acid and/or a salt thereof.

Sum up these, the copolymer containing the substituted alkene as a constituent unit may be a copolymer containing vinyl chloride and a water-soluble ethylenically unsaturated monomer as constituent units, may be a copolymer containing vinyl chloride and (meth)acrylic acid and/or a salt thereof as a constituent unit, may be a copolymer containing vinyl chloride and (meth)acrylic acid as constituent units, and may be a copolymer (acrylic acid-vinyl chloride copolymer) containing vinyl chloride and acrylic acid as constituent units.

The copolymer containing the unsubstituted alkene as a constituent unit may be a copolymer containing ethylene and a water-soluble ethylenically unsaturated monomer as constituent units, may be a copolymer containing ethylene and (meth)acrylic acid and/or a salt thereof as a constituent unit, may be a copolymer containing ethylene and an acrylic acid salt as constituent units, may be a copolymer containing ethylene and sodium acrylate or potassium acrylate as constituent units (ethylene-sodium acrylate copolymer or ethylene-potassium acrylate copolymer), and may be an ethylene-sodium acrylate copolymer.

The homopolymer containing a substituted or unsubstituted alkene as a constituent unit may be a homopolymer containing a substituted alkene as a constituent unit, may be a homopolymer containing a halogenated alkene and/or an alkene substituted with an aromatic substituent (for example, phenyl group or naphthyl group) as a constituent unit, may be a homopolymer containing vinyl halide (for example, chloroethylene, bromoethylene, or fluoroethylene) and/or ethylene substituted with a phenyl group (styrene) as a constituent unit, and may be polyvinyl chloride or polystyrene, which is a homopolymer of vinyl halide (particularly, chloroethylene).

From the viewpoint that the permeation rate is further improved and the viewpoint that the re-wet after liquid absorption is further suppressed, the content of the compound A may be 5 parts by mass or more, 10 parts by mass or more, 15 parts by mass or more, 35 parts by mass or more, 55 parts by mass or more, 75 parts by mass or more, 85 parts by mass or more, or 95 parts by mass or more, with respect to 100 parts by mass in the total amount of the compound A and the compound B. The content of the compound A may be less than 100 parts by mass, or 95 parts by mass or less with respect to 100 parts by mass in the total amount of the compound A and the compound B. In the coated resin particle according to the present embodiment, the content of the compound A with respect to 100 parts by mass in the total amount of the compound A and the compound B can be regarded as an area of a region where the compound A is present in the coating layer with respect to the total area of the coating layer.

From the viewpoint that the permeation rate is further improved, the content of the compound A may be 0.1 parts by mass or more, 1.0 part by mass or more, 5.0 parts by mass or more, 10 parts by mass or more, 15 parts by mass or more, 20 parts by mass or more, or 25 parts by mass or more, with respect to 100 parts by mass of the water-absorbent resin particle. From the viewpoint that the re-wet after liquid absorption is further suppressed, the content of the compound A may be 40 parts by mass or less, 30 parts by mass or less, 20 parts by mass or less, 15 parts by mass or less, or 10 parts by mass or less, with respect to 100 parts by mass of the water-absorbent resin particle.

The compound B is a compound that is more water-soluble than the compound A. A dissolved content of the compound B in 100 g of water at 25°C may be 20% by mass or more, 30% by mass or more, 40% by mass or more, 50% by mass or more, 60% by mass or more, 70% by mass or more, or 80% by mass or more. The dissolved content of the compound B in 100 g of water at 25°C may be 100% by mass or less, 95% by mass or less, 90% by mass or less, or 85% by mass or less. The dissolved content of the compound B is measured by a method described in Examples described later.

From the viewpoint that the re-wet after liquid absorption is further suppressed and the viewpoint that the permeation rate is further improved, a dissolved content of the compound B in 100 g of water at 25°C may be higher than the dissolved content of the compound A by 20% by mass or more. From the viewpoint that the re-wet after liquid absorption is further suppressed and the viewpoint that the permeation rate is further improved, a difference (B - A) between the dissolved content of the compound B and the dissolved content of the compound A may be 25% by mass or more, 30% by mass or more, 35% by mass or more, 40% by mass or more, 45% by mass or more, 50% by mass or more, 55% by mass or more, 60% by mass or more, 65% by mass or more, 70% by mass or more, 75% by mass or more, or 80% by mass or more. The difference (B - A) between the dissolved content of the compound B and the dissolved content of the compound A may be, for example, 100% by mass or less, 95% by mass or less, 90% by mass or less, or 85% by mass or less.

The compound B may be at least one compound selected from the group consisting of an inorganic salt and an organic compound having a hydrophilic group (hereinafter, simply referred to as a "hydrophilic group-containing compound").

Examples of the inorganic salt include sodium chloride, potassium chloride, sodium carbonate, sodium hydrogen carbonate, and the like.

The hydrophilic group which the hydrophilic group-containing compound may have may be, for example, a hydroxy group, an oxyalkylene group, a carboxyl group, an amino group, and the like. Appropriate adjustment of the type and number of the hydrophilic groups enables the dissolved content of the compound B to be adjusted.

Examples of the hydrophilic group-containing compound include a polymer containing an alkylene oxide as a constituent unit, and fructose.

The polymer containing the alkylene oxide as a constituent unit may be a polyalkylene oxide (homopolymer) containing only one alkylene oxide as a constituent unit, may be a polyalkylene oxide (copolymer) containing two or more alkylene oxides as constituent units, or may be a copolymer containing one or two or more alkylene oxides and a monomer component other than an alkylene oxide as constituent units. The polymer containing the alkylene oxide as a constituent unit may be a polyalkylene oxide containing only one alkylene oxide as a constituent unit.

Examples of the alkylene oxide include ethylene oxide and propylene oxide. The alkylene oxide may be ethylene oxide.

Sum up these, the polymer containing the alkylene oxide as a constituent unit may be at least one selected from the group consisting of a homopolymer (polyethylene glycol) containing ethylene oxide as a constituent unit and an ethylene-propylene copolymer containing ethylene oxide and propylene oxide as constituent units, or may be polyethylene glycol.

The number-average molecular weight of the polymer containing the alkylene oxide as a constituent unit may be 100 to 200,000 or 5,000 to 20,000.

From the viewpoint that ther re-wet after liquid absorption is further suppressed, the content of the compound B may be 95 parts by mass or less, 85 parts by mass or less, 60 parts by mass or less, 35 parts by mass or less, 10 parts by mass or less, 5 parts by mass or less, or 3 parts by mass or less, with respect to 100 parts by mass in the total amount of the compound A and the compound B. From the viewpoint that the permeation rate is further improved, the content of the compound B may be 0.1 parts by mass or more, 0.5 parts by mass or more, 1.0 part by mass or more, or 2.0 parts by mass or more, with respect to 100 parts by mass in the total amount of the compound A and the compound B. The content of the compound B may be, for example, 1 to 85 parts by mass. In the coated resin particle according to the present embodiment, the content of the compound B with respect to 100 parts by mass in the total amount of the compound A and the compound B can be regarded as an area of a region where the compound B is present in the coating layer with respect to the total area of the coating layer.

From the viewpoint that the re-wet after liquid absorption is further suppressed, the content of the compound B may be 30 parts by mass or less, 25 parts by mass or less, 15 parts by mass or less, 10 parts by mass or less, 5 parts by mass or less, or 1 part by mass or less, with respect to 100 parts by mass of the water-absorbent resin particle. From the viewpoint that the permeation rate is further improved, the content of the compound B may be 0.01 parts by mass or more, 0.1 parts by mass or more, 0.5 parts by mass or more, 1.0 part by mass or more, 2.0 parts by mass or more, or 2.5 parts by mass or more, with respect to 100 parts by mass of the water-absorbent resin particle.

The coating layer may have a sea-island structure composed of a sea part including one compound of the compound A and the compound B, and an island part including the other compound. The island part may be dispersed in the sea part. The sea-island structure can be confirmed by an analysis method such as Raman spectrometric method. For example, when the coating layer is formed, the sea-island structure is easily formed by using a coating material containing the compound A in an emulsion state.

The sea-island structure may be formed of the sea part including the compound A and the island part including the compound B. In this case, a state in which the water-absorbent resin particles are restricted by the compound A constituting the sea part is maintained to some extent even though the entire compound B is dissolved. Therefore, the effect of suppressing re-wet after liquid absorption and the effect of improving the permeation rate is better.

In the coated resin particle in which the coating layer has the sea-island structure, since a part corresponding to a water channel, in which the compound B is present, is localized, the water channel that corresponds to the starting point of water absorption is larger as compared to a case where the compound B is localized and does not form the island part, and the water absorption can be initiated more rapidly after a certain delay in water absorption. Therefore, it is considered that a blocking phenomenon and re-wet after liquid absorption are less likely to occur when the water-absorbent resin particles are used for an absorbent article, resulting in the improvement of the liquid absorption performance (the permeation rate and the effect of suppressing re-wet).

The region including the compound B (hereinafter, referred to as a compound B-containing region) extends in a thickness direction of the coating layer. The compound B-containing region is a sea part or an island part, and may be an island part. At least a part of the compound B-containing region may constitute half or more of the thickness of the coating layer including the region, and at least a part of the compound B-containing region may constitute the entire thickness of the coating layer including the region. The entire compound B-containing region may constitute half or more of the thickness of the coating layer including the region, and may constitute the entire thickness of the coating layer including the region. Since the compound B contained in the compound B-containing region (the sea part or the island part) is more water-soluble than the compound A, a part of a surface of the water-absorbent resin particle corresponding to the compound B-containing region can come into contact with the liquid earlier than a part of the surface of the water-absorbent resin particle corresponding to the compound A-containing region (the sea part or the island part) because of dissolution of the compound B. That is, although the contact of the water-absorbent resin particle with the liquid is blocked by the coating layer, the water channel, which is formed by dissolution of the compound B-containing region (the sea part or the island part) after the lapse of a predetermined time from when the water-absorbent resin particle comes into contact with the liquid, allows the water-absorbent resin particle to come into contact with the liquid (enables water absorption).

The coated resin particle according to the present embodiment can control a water absorption inflection point which is the time until the water absorption rate sharply increases. The water absorption inflection point can be adjusted by controlling, for example, the total content of the coating layer with respect to 100 parts by mass of the water-absorbent resin particle, a ratio between the sea part and the island part, and a type of compounds used for each of the sea part and the island part (for example, in a case where the sea part includes a copolymer containing ethylene and (meth)acrylate as constituent units, the degree of neutralization and/or the type of counterions of the copolymer and the like).

The water absorption inflection point of the coated resin particle according to the present embodiment may be, for example, 1.5 minutes or more, 2.0 minutes or more, 2.5 minutes or more, 3.5 minutes or more, 4.0 minutes or more, 4.5 minutes or more, 5.0 minutes or more, or 5.5 minutes or more, and may be 15.0 minutes or less, 10.0 minutes or less, 7.0 minutes or less, or 6.5 minutes or less. The water absorption inflection point of the coated resin particle according to the present embodiment may be, for example, 1.5 to 15.0 minutes, 1.5 to 10.0 minutes, 1.5 to 7.0 minutes, or 2.0 to 6.5 minutes. The water absorption inflection point of the coated resin particle is measured by a method described in Examples described later.

The total content of the coating layer may be 3 parts by mass or more, 5 parts by mass or more, 10 parts by mass or more, 15 parts by mass or more, 20 parts by mass or more, or 25 parts by mass or more with respect to 100 parts by mass of the water-absorbent resin particle. The total content of the coating layer may be 50 parts by mass or less, 45 parts by mass or less, 40 parts by mass or less, or 35 parts by mass or less with respect to 100 parts by mass of the water-absorbent resin particle. The total content of the coating layer may be 3 to 50 parts by mass, or 10 to 40 parts by mass with respect to 100 parts by mass of the water-absorbent resin particle. The total content of the coating layer referred to herein corresponds to the use amount of the coating material. In the coated resin particle according to the present embodiment, the water absorption inflection point tends to extend as the amount of the coating layer is larger with respect to the water-absorbent resin particle.

The content of the island part may be, for example, 20 parts by mass or less, 16 parts by mass or less, or 12 parts by mass or less with respect to 100 parts by mass of the content of the sea part. The content of the island part may be, for example, 1 part by mass or more, 5 parts by mass or more, or 8 parts by mass or more with respect to 100 parts by mass of the content of the sea part. The content of the island part may be, for example, 1 part by mass or more and 20 parts by mass or less, 5 parts by mass or more and 16 parts by mass or less, or 8 parts by mass or more and 12 parts by mass or less with respect to 100 parts by mass of the content of the sea part. In the coated resin particle according to the present embodiment, as the content of the island part with respect to the content of the sea part is small, the water absorption inflection point tends to extend. Furthermore, as the content of the island part with respect to the content of the sea part is large, the water absorption inflection point tends to be shortened.

In a case where the sea part includes the copolymer containing ethylene and (meth)acrylate as constituent units, a degree of neutralization of a carboxy group in the copolymer constituting the sea part may be 70% by mol or more, 75% by mol or more, 80% by mol or more, 85% by mol or more, 90% by mol or more, or 95% by mol or more of the carboxy group in the (meth)acrylate contained as a constituent unit. The degree of neutralization may be 80 to 100% by mol or 85 to 100% by mol of the carboxy group in (meth)acrylate contained as a constituent unit. In the coated resin particle according to the present embodiment, in a case where the sea part includes the copolymer containing ethylene and (meth)acrylate as constituent units, the water absorption inflection point tends to extend as a degree of neutralization of the copolymer is high.

In a case where the coated resin particle, which contains, as the sea part, the copolymer containing ethylene and (meth)acrylate as constituent units, contains a copolymer including a sea part in which a salt (counterion) is a potassium ion, the water absorption inflection point tends to extend as compared to a case of containing a copolymer including a sea part in which a salt is a sodium ion.

(Inorganic Particle)

The coated resin particle may be used in combination with an inorganic particle. Examples of the inorganic particle include a silica particle such as amorphous silica. The amount of the inorganic particles may be 0.05% by mass or more, 0.1% by mass or more, 0.15% by mass or more, or 0.2% by mass or more, and may be 5.0% by mass or less, 3.0% by mass or less, 1.0% by mass or less, 0.5% by mass or less, or 0.3% by mass or less, based on the mass of polymer particles. The average particle diameter of the inorganic particles may be 0.1 to 50 $\mu$m, 0.5 to 30 $\mu$m, or 1 to 20 $\mu$m. The average particle diameter can be measured by a pore electric resistance method or a laser diffraction/scattering method depending on the characteristics of particles.

The coated resin particles of the present embodiment can be used alone, and also can be used as mixed particles by mixing with a resin particle having a water absorption property other than the coated resin particle (hereinafter, simply referred to as "other resin particle").

[Water-absorbent Resin Composition]

The water-absorbent resin composition according to one embodiment contains the above-mentioned coated resin particle, and a resin particle (other resin particle) having a water absorption property other than the coated resin particle. Using the water-absorbent resin composition enables the delay of time for allowing the coated resin particle to reach the swelling state as compared to the case of using the other resin particle alone. As a result, the occurrence of the gel blocking phenomenon can be suppressed. In addition, in a case of using the water-absorbent resin composition according to the present embodiment, any water absorption behavior may be achieved by appropriately changing a type of the coated resin particles, a type of the other resin particles, a mixing ratio of the coated resin particles and the other resin particles, and the like.

In the water-absorbent resin composition, the content of the coated resin particles may be, for example, 5 parts by mass or more or 15 parts by mass or more with respect to 100 parts by mass in the total content of the coated resin particles and the other resin particles, and may be 95 parts by mass or less, 85 parts by mass or less, 60% by mass or less, 40% by mass or less, 30% by mass or less, or 25% by mass or less.

The occurrence of the gel blocking phenomenon is suppressed in the coated resin particle of the present invention. Therefore, in a case where the mixed particles obtained by mixing the coated resin particles with the other resin particles are used in the absorber, the diffusibility of the liquid in the absorber can be improved, that is, the resin particles in the absorber can be used efficiently as compared to a case where the other resin particles are used alone. As a result, the use amount of the resin particles in the absorber can be reduced as compared to the case of using the other resin particles alone.

In addition, in the absorber, hydrophilic fibers such as pulp may be mixed with the other resin particles for the purpose of improving the diffusibility of the liquid, but the use amount of the hydrophilic fibers is required to be reduced from the viewpoint of environmental protection. However, when the use amount of the hydrophilic fibers in the absorber is reduced, the proportion of the other resin particles is increased, resulting in that the gel blocking phenomenon is prone to occur. Regarding this point, when the coated resin particles of the present invention are used in combination with the other resin particles, the occurrence of the gel blocking phenomenon can be suppressed even though the use amount of the hydrophilic fibers is reduced.

In the other resin particles, a lower limit value of the water retention capacity with respect to physiological saline (0.9% by mass sodium chloride aqueous solution) may be 25 g/g or more, may be 30 g/g or more, or may be 35 g/g or more. In addition, an upper limit value of the water retention capacity may be 55 g/g or less, may be 50 g/g or less, or may be 45 g/g or less. The water retention capacity can be measured by a method described in Examples described later.

The water-absorbent resin particle constituting the coated resin particle (target to be coated with the coating layer) may be the same particle as the other resin particle, or may be a particle different from the other resin particle. The water-absorbent resin particle constituting the coated resin particle may be the same particle as the other resin particle.

[Method For Producing Coated Resin Particle]

The coated resin particle according to the present embodiment is produced by a method including a step of coating at least a part of the water-absorbent resin particle with a coating material to form a coating layer on at least a part of a surface of the water-absorbent resin particle. The coating material contains the compound A and the compound B more water-soluble than the compound A. Details of the compound A and the compound B are as mentioned above. According to the method for producing a coated resin particle of the present embodiment, it is possible to obtain the coated resin particle that can improve a permeation rate of the liquid while sufficiently suppressing an increase of the re-wet amount after liquid absorption for the practical purpose as compared to the case of using a conventional water-absorbent resin particle alone, by using the conventional water-absorbent resin particle in combination when the coated resin particle is used in the absorbent article. In addition, according to the method for producing a coated resin particle of the present embodiment, it is possible to easily obtain the coated resin particle with the water absorption inflection point controlled. In the method for producing a coated resin particle according to the present embodiment, the aspects described in the above-mentioned Coated Resin Particle can be applied without limitation.

The coating material can be prepared by mixing the compound A, the compound B, and a liquid medium. In the coating material, the compound A is in a solution state or an emulsion state. The coating material containing the compound A in a solution state can be obtained by mixing the compound A, the compound B, and the liquid medium. The coating material containing the compound A in the emulsion state can be obtained by, for example, mixing an emulsion containing particles, which contain the compound A and are dispersed in the liquid medium, and the compound B. The emulsion can be formed by the application of a general emulsifying method. In the coating material, the compound B may be dissolved or dispersed in the liquid medium.

As the liquid medium, a solvent removeable by drying may be used. Examples of the liquid medium include water, ether (for example, tetrahydrofuran), acetone, methanol, ethanol, and hydrocarbon (hexane, heptane, and the like).

Concentrations of the compound A and the compound B in the coating material can be appropriately adjusted in consideration of the coating layer having a target thickness, the amount of the water-absorbent resin particle to be coated, and the like. A content of the compound A in the coating material may be, for example, 1% to 50% by mass, 3% to 30% by mass, or 5% to 20% by mass, based on the total amount of the coating material. A content of the compound B in the coating material may be, for example, 0.1% to 30% by mass, 0.5% to 20% by mass, or 1% to 10% by mass, based on the total amount of the coating material.

The content of the compound B in the coating material may be, for example, 1 to 500 parts by mass with respect to 100 parts by mass of the content of the compound A. The lower limit of the content of the compound B with respect to 100 parts by mass of the content of the compound A may be 5 parts by mass or more, or 8 parts by mass or more. The upper limit of the content of the compound B with respect to 100 parts by mass of the content of the compound A may be, for example, 400 parts by mass or less, 300 parts by mass or less, 200 parts by mass or less, 100 parts by mass or less, 50 parts by mass or less, 25 parts by mass or less, or 15 parts by mass or less.

The coating layer can be formed by (1) a method for adding the coating material to a hydrocarbon dispersion medium in which the water-absorbent resin particles are dispersed, (2) a method of adding the coating material and the water-absorbent resin particles in the hydrocarbon dispersion medium substantially at the same time, or (3) a method for bringing the coating material into contact with the water-absorbent resin particles in a dry state, for example. Hereinafter, each method will be specifically described.

An example of the above-mentioned method (1) will be described. First, a separable flask equipped with a reflux cooling device, a dropping funnel, a nitrogen gas introduction tube, and a stirrer is prepared. Subsequently, the hydrocarbon dispersion medium and the water-absorbent resin particles are injected into the separable flask, and the resultant mixture is sufficiently stirred while maintaining a high temperature (for example, 60°C to 80°C). The coating material is added into the above-mentioned separable flask and sufficiently stirred, and thereafter, the separable flask is immersed in an oil bath set at a high temperature (for example, 100°C to 125°C), and water that may be included in the reaction system is extracted to the outside of the system while refluxing the hydrocarbon dispersion medium by azeotropic distillation of the hydrocarbon dispersion medium and water. Thereafter, the hydrocarbon dispersion medium is evaporated to obtain coated resin particles containing water-absorbent resin particles whose surfaces are coated with the coating material.

An example of the above-mentioned method (2) will be described. First, a separable flask equipped with a reflux cooling device, a dropping funnel, a nitrogen gas introduction tube, and a stirrer is prepared. Subsequently, the hydrocarbon dispersion medium, the water-absorbent resin particles, and the coating material are injected into the separable flask, and the resultant mixture is sufficiently stirred while maintaining a high temperature (for example, 60°C to 80°C). Thereafter, the hydrocarbon dispersion medium is evaporated to obtain coated resin particles containing water-absorbent resin particles whose surfaces are coated with the coating material.

Although there are various methods for the above-mentioned method (3), (3-1) a method using an eggplant flask, (3-2) a method using a sprayer, and (3-3) a method using various granulators will be described below as representative examples thereof.

(3-1)
The coating material is injected into the eggplant flask, and subsequently the water-absorbent resin particles are injected thereto. The eggplant flask is attached to an evaporator, and heated while rotating to distill off a liquid medium contained in the coating material under reduced pressure conditions. Thus, coated resin particles containing water-absorbent resin particles whose surfaces are coated with the coating material are obtained.

(3-2)

The water-absorbent resin particles are added to a separable flask equipped with a stirrer blade, and stirred. The coating material is sprayed on the water-absorbent resin particles whirled up by stirring with the stirrer blade. Spraying of the coating material can be performed using a two-fluid nozzle, for example. It is desirable that the coating material is atomized by a stream of an inert gas such as nitrogen and sprayed, because then uniform coating can be expected. Thereafter, the contents of the separable flask are taken out, heated by a hot-air dryer, and thereafter cooled to room temperature to obtain coated resin particles.

(3-3)

Examples of the granulators used for producing the coated resin particles include a tumbling granulator, a stirring granulator, and a fluid bed granulator.

When the tumbling granulator is used, an inclined shallow circular container equipped in the tumbling granulator is rotated, the water-absorbent resin particles are supplied to the circular container, and an appropriate amount of the coating material is also added. Then, while some of the water-absorbent resin particles aggregate, the coating layer is formed on a surface thereof by the solvent or dispersion medium contained in the coating material during rotating. A step of adding the water-absorbent resin particles and the coating material may be performed a plurality of times as necessary.

When the stirring granulator is used, the water-absorbent resin particles are injected into a mixer equipped in the stirring granulator, and the coating material is added while performing the mixing by stirring. Then, while some of the water-absorbent resin particles aggregate, the coating layer is formed on a surface thereof by the liquid medium contained in the coating material during stirring. A step of adding the water-absorbent resin particles and the coating material may be performed a plurality of times as necessary. Excessive aggregation of the water-absorbent resin particles may be suppressed by controlling the shearing force of the mixer.

When the fluid bed granulator is used, first, the water-absorbent resin particles are injected into a container that is equipped in the fluid bed granulator and can blow hot air from the lower part to previously fluidize the water-absorbent resin particles. Thereafter, when the coating material is scattered from a nozzle equipped in the container, while some of the water-absorbent resin particles aggregate, the coating layer is formed on a surface thereof by the liquid medium contained in the coating material during stirring. The coating material may be scattered a plurality of times as necessary. Excessive aggregation of the water-absorbent resin particles may be suppressed by adjusting the scattering amount and the scattering frequency of the coating material. As the fluid bed granulator, for example, a fluid bed granulator FBD/SG (manufactured by YENCHEN MACHINERY CO., LTD.) can be used.

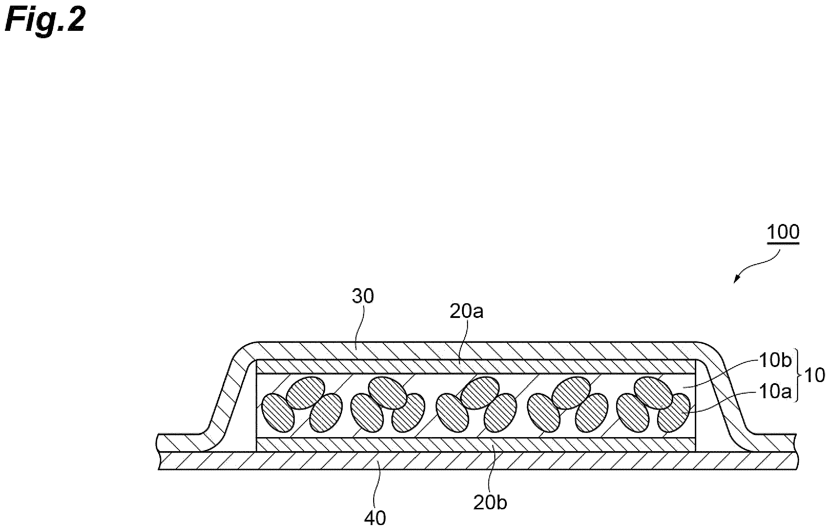

The coated resin particle is used to form an absorber constituting an absorbent article such as diapers, for example. Fig. 2 is a cross-sectional view showing an example of an absorbent article. An absorbent article 100 shown in Fig. 2 includes a sheet shaped absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 2, there is a portion shown so that a gap is present between members, but the members may be in close contact with each other without the gap.

The absorber 10 has coated resin particles 10a according to the above-mentioned present embodiment and a fiber layer 10b containing a fibrous material. The coated resin particles 10a are dispersed in the fiber layer 10b.

The core wrap 20a is disposed on one surface side of the absorber 10 (upper side of the absorber 10 in Fig. 1) in a state of being in contact with the absorber 10. The core wrap 20b is disposed on the other surface side of the absorber 10 (on a lower side of the absorber 10 in Fig. 1) in a state of being in contact with the absorber 10. The absorber 10 is disposed between the core wrap 20a and the core wrap 20b. Examples of the core wraps 20a and 20b include tissues, non-woven fabrics, and the like. The core wrap 20a and the core wrap 20b each have, for example, a main surface having the same size as that of the absorber 10.

The liquid permeable sheet 30 is disposed on the outermost part at the side where the liquid to be absorbed enters. The liquid permeable sheet 30 is disposed on the core wrap 20a in a state of being in contact with the core wrap 20a. Examples of the liquid permeable sheet 30 include a non-woven fabric made of a synthetic resin such as polyethylene, polypropylene, polyester, and polyamide, and a porous sheet. The liquid impermeable sheet 40 is disposed on the outermost part at the opposite side to the liquid permeable sheet 30 in the absorbent article 100. The liquid impermeable sheet 40 is disposed on the lower side of the core wrap 20b in a state of being in contact with the core wrap 20b. Examples of the liquid impermeable sheet 40 include a sheet made of a synthetic resin such as polyethylene, polypropylene, and polyvinyl chloride, and a sheet made of a composite material of these synthetic resins and a non-woven fabric. The liquid permeable sheet 30 and the liquid impermeable sheet 40 each have, for example, a main surface wider than the main surface of the absorber 10, and outer edges of the liquid permeable sheet 30 and the liquid impermeable sheet 40 each extend around the absorber 10 and the core wraps 20a and 20b.

The magnitude relationship between the absorber 10, the core wraps 20a and 20b, the liquid permeable sheet 30, and the liquid impermeable sheet 40 is not particularly limited, and is appropriately adjusted according to the use of the absorbent article or the like. In addition, the method of retaining the shape of the absorber 10 using the core wraps 20a and 20b is not particularly limited, and as shown in Fig. 2, the absorber may be wrapped by a plurality of core wraps, and the absorber is wrapped by one core wrap.

The absorber 10 may further include resin particles (the other resin particles) having a water absorption property other than the coated resin particles 10a. In a case where the other resin particles are included, the content of the coated resin particles 10a may be 5 parts by mass or more or 15 parts by mass or more, and may be 95 parts by mass or less or 85 parts by mass or less with respect to 100 parts by mass in the total content of the coated resin particles and the other resin particles, for example.

## EXAMPLES

Hereinafter, the present invention will be more specifically described with reference to examples. However, the present invention is not limited to these examples.

1. Production of Coated Resin Particle

[Comparative Example 1]

A round-bottomed cylindrical separable flask with an inner diameter of 11 cm and a volume of 2 L equipped with a reflux cooling device, a dropping funnel, a nitrogen gas introduction tube, and a stirrer (a stirrer blade having two stages of four inclined paddle blades with a blade diameter of 5 cm) was prepared. 293 g of n-heptane (hydrocarbon dispersion medium) and 0.736 g of a maleic anhydride-modified ethylene/propylene copolymer (polymeric dispersant, manufactured by Mitsui Chemicals, Inc., Hi-Wax 1105A) were added into this separable flask to obtain a mixture. The dispersant was dissolved by heating to 80°C while stirring this mixture at a rotation speed of 300 rpm. Thereafter, the mixture was cooled to 55°C.

Subsequently, 92.0 g of a 80.5% by mass acrylic acid aqueous solution (acrylic acid: 1.03 mol) was put into a triangular flask having a volume of 500 mL. Subsequently, while cooling from the outside, 102.2 g of a 30% by mass sodium hydroxide aqueous solution was added dropwise to neutralize 75% by mol of acrylic acid. Thereafter, 0.092 g of hydroxyethyl cellulose (thickener, manufactured by SUMITOMO SEIKA CHEMICALS CO., LTD., HEC AW-15F), 0.0736 g (0.272 mmol) of potassium persulfate (water-soluble radical polymerization initiator), and 0.0101 g (0.0580 mmol) of ethylene glycol diglycidyl ether (internal crosslinking agent), and 32.85 g of ion-exchanged water were added, and then dissolved to prepare a first stage monomer aqueous solution.

The above-mentioned first stage monomer aqueous solution was added to the above-mentioned separable flask, and stirring was then carried out for 10 minutes. 0.736 g of sucrose stearic acid ester (surfactant, Mitsubishi-Chemical Foods Corporation, RYOTO Sugar Ester S-370, HLB value: 3) was heat-dissolved in 6.62 g of n-heptane by heating to obtain a surfactant solution. 7.356 g of the obtained surfactant solution was added to the separable flask to obtain a reaction solution. Then, the inside of the separable flask system was sufficiently replaced with nitrogen while stirring the reaction solution at the rotation speed of 550 rpm. Thereafter, the above-mentioned separable flask was immersed in a water bath at 70°C to raise the temperature of the reaction solution, and a first stage polymerization was performed for 10 minutes to obtain a first stage reaction mixture.

Subsequently, 128.8 g of a 80.5% by mass acrylic acid aqueous solution (acrylic acid: 1.44 mol) was put into another triangular flask having a volume of 500 mL. Subsequently, while cooling from the outside, 143.1 g of a 30% by mass sodium hydroxide aqueous solution was added dropwise to neutralize 75% by mol of acrylic acid. Thereafter, 0.1030 g (0.3810 mmol) of potassium persulfate, 0.0116 g (0.0666 mmol) of ethylene glycol diglycidyl ether (internal crosslinking agent), and 0.63 g of ion-exchanged water were added and then dissolved to prepare a second stage monomer aqueous solution.

The first stage reaction mixture was cooled to 25°C while stirring the first stage reaction mixture at a rotation speed of 1000 rpm, and the total amount of the second stage monomer aqueous solution was then added to the first stage reaction mixture to obtain a reaction solution. The inside of the system was sufficiently replaced with nitrogen while stirring the reaction solution. Thereafter, the separable flask was immersed in a water bath at 70°C to raise the temperature of the reaction solution, and the second stage polymerization was performed for 5 minutes to obtain a second stage reaction mixture (polymer particles before surface crosslinking).

After the second stage polymerization, the temperature of the second stage reaction mixture was raised in an oil bath at 125°C, and 254 g of water was extracted to the outside of the system while refluxing n-heptane by azeotropic distillation of n-heptane and water. Subsequently, 0.0884 g (0.5075 mmol) of ethylene glycol diglycidyl ether was added as a surface crosslinking agent, and the mixture was then maintained at 83°C for 2 hours to obtain a dispersion liquid of the polymer particles after surface crosslinking.

Thereafter, the temperature of the dispersion liquid of the polymer particles after the above-mentioned surface crosslinking was raised in an oil bath at 125°C, and n-heptane was evaporated and dried to obtain a dried product. This dried product was passed through a sieve having an opening of 850 $\mu$m to obtain 233.4 g of water-absorbent resin particles (1) in a state in which spherical particles aggregated and were not coated.

Thereafter, 200 g of the above-mentioned water-absorbent resin particles (1) and 0.4 g of TOKUSIL (Oriental Silicas Corporation, product number: NP-S), which is hydrophilic silica, were put into a SUS bottle, and mixed for 30 minutes by a cross rotary mixer to obtain water-absorbent resin particles (2).

The water retention capacity of the water-absorbent resin particles (1) and the water-absorbent resin particles (2) produced in Comparative Example 1 was 40 g/g. The water retention capacity was measured by a method described below. After 500 g of physiological saline was added to a 500 mL polyethylene beaker, 2.00 g of the water-absorbent resin particles were added in small portions while allowing rotation at a rotation speed of 600 rpm using a stirrer. After addition of the total amount of the water-absorbent resin particles was completed, stirring was carried out for 30 minutes. Subsequently, after the mixture was transferred into a cotton bag (Cotton broadcloth No. 60, a width of 100 mm × a length of 200 mm), the upper part of the cotton bag was closed with a rubber band. Subsequently, centrifugation (167 G) was performed for 1 minute using a centrifuge (manufactured by KOKUSAN Co., Ltd., product number: H-122), and a mass WA of the cotton bag containing the swollen gel after dehydration was then measured. The same operation was performed without putting the water-absorbent resin particles into the cotton bag, and a mass WB of the empty cotton bag was measured. Then, the water retention capacity (25°C) of the physiological saline was calculated by the following equation.

$$\text{Water retention capacity [g/g]} = (WA - WB)/\text{mass of water-absorbent resin particles} (= 2.00)$$

[Example 1]

The operations of Comparative Example 1 before the addition of TOKUSIL were repeated, the collected water-absorbent resin particles (1) were classified with a comb having an opening of 250 $\mu$m, and 500 g or more of the water-absorbent resin particles having a particle diameter of 250 to 850 $\mu$m was obtained.

10.0 g of a 25% aqueous dispersion emulsion of an ethylene-sodium acrylate copolymer (SUMITOMO SEIKA CHEMICALS CO., LTD., ZAIKTHENE N) serving as the compound A, and 0.25 g of a polyethylene glycol (Tokyo Chemical Industry Co., Ltd., PEG 6000 molecular weight: 7300 to 9300) serving as the compound B were diluted with 14.75 g of ion-exchanged water in a polybeaker having an internal volume of 1 L, to prepare a coating material A.

500.0 g of the water-absorbent resin particles was injected to a container of the fluid bed granulator, and hot air at 50°C was blown from the lower part of the container. Subsequently, while drying 25 g of the coating material, the coating material A was sprayed on the water-absorbent resin particles whirled up by blowing. After the coating material was sprayed, the water-absorbent resin particles were dried at 50°C for 30 minutes. After the water-absorbent resin particles were dried, 502.3 g of coated resin particles was obtained.

50.0 g of the obtained coated resin particles was spread on a metal tray having a length of 26 cm and a width of 20 cm, and a lid made of an aluminum foil was put thereon. The aluminum foil was perforated, and the coated resin particles were heated for 60 minutes by a hot-air dryer (ADVANTEC, FV-320) set at 80°C to obtain 50.0 g of coated resin particles.

[Example 2]

40.0 g of a 25% aqueous dispersion emulsion of an ethylene-sodium acrylate copolymer (SUMITOMO SEIKA CHEMICALS CO., LTD., ZAIKTHENE N) serving as the compound A, and 1.0 g of a polyethylene glycol (Tokyo Chemical Industry Co., Ltd., PEG 6,000) serving as the compound B were diluted with 59.0 g of ion-exchanged water, to prepare a coating material B. 50.0 g of coated resin particles was obtained in the same manner as in Example 1, except that the coating material B was used instead of the coating material A.

[Example 3]

600.0 g of a 25% aqueous dispersion emulsion of an ethylene-sodium acrylate copolymer (SUMITOMO SEIKA CHEMICALS CO., LTD., ZAIKTHENE N) serving as the compound A, and 15.0 g of a polyethylene glycol (Tokyo Chemical Industry Co., Ltd., PEG 6,000) serving as the compound B were diluted with 885.0 g of ion-exchanged water, to prepare a coating material C. 50.0 g of coated resin particles was obtained in the same manner as in Example 1, except that the coating material C was used instead of the coating material A.

[Example 4]

200.0 g of a 25% aqueous dispersion emulsion of an ethylene-sodium acrylate copolymer (SUMITOMO SEIKA CHEMICALS CO., LTD., ZAIKTHENE N) serving as the compound A, and 0.5 g of a polyethylene glycol (Tokyo Chemical Industry Co., Ltd., PEG 6,000) serving as the compound B were diluted with 299.5 g of ion-exchanged water, to prepare a coating material D. 50.0 g of coated resin particles was obtained in the same manner as in Example 1, except that the coating material D was used instead of the coating material A.

[Example 5]

200.0 g of a 25% aqueous dispersion emulsion of an ethylene-sodium acrylate copolymer (SUMITOMO SEIKA CHEMICALS CO., LTD., ZAIKTHENE N) serving as the compound A, and 5.0 g of a polyethylene glycol (Tokyo Chemical Industry Co., Ltd., PEG 6,000) serving as the compound B were diluted with 295.0 g of ion-exchanged water, to prepare a coating material E. 50.0 g of coated resin particles was obtained in the same manner as in Example 1, except that the coating material E was used instead of the coating material A.

[Example 6]

20.0 g of a 25% aqueous dispersion emulsion of an ethylene-sodium acrylate copolymer (SUMITOMO SEIKA CHEMICALS CO., LTD., ZAIKTHENE N) serving as the compound A, and 25.0 g of a polyethylene glycol (Tokyo Chemical Industry Co., Ltd., PEG 6,000) serving as the compound B were diluted with 455.0 g of ion-exchanged water, to prepare a coating material F. 50.0 g of coated resin particles was obtained in the same manner as in Example 1, except that the coating material F was used instead of the coating material A.

[Example 7]

20.0 g of a 25% aqueous dispersion emulsion of an ethylene-sodium acrylate copolymer (SUMITOMO SEIKA CHEMICALS CO., LTD., ZAIKTHENE N) serving as the compound A, and 50.0 g of a polyethylene glycol (Tokyo Chemical Industry Co., Ltd., PEG 6,000) serving as the compound B were diluted with 430.0 g of ion-exchanged water, to prepare a coating material G. 50.0 g of coated resin particles was obtained in the same manner as in Example 1, except that the coating material G was used instead of the coating material A.

[Example 8]

Polymer particles (aqueous solution polymer, median particle diameter: 408 $\mu$m) collected from a commercially available diaper sold in Japan (manufactured by Kao Corporation, trade name: Merries Pants Smooth Air-through, S size) were used as a target to be coated, and 200.0 g of a 25% aqueous dispersion emulsion of an ethylene-sodium acrylate copolymer (SUMITOMO SEIKA CHEMICALS CO., LTD., ZAIKTHENE N) serving as the compound A, and 5.0 g of a polyethylene glycol (Tokyo Chemical Industry Co., Ltd., PEG 6,000) serving as the compound B were diluted with 295.0 g of ion-exchanged water, to prepare a coating material H. 50.0 g of coated resin particles was obtained in the same manner as in Example 1, except that the coating material H was used instead of the coating material A. The water retention capacity of the polymer particles collected from the diaper was 31 g/g.

[Example 9]

<Production of Compound A>

Water and ice were added to a plastic tray having a length of 27 cm, a width of 38 cm, and a depth of 7 cm to produce an ice bath at 3°C. A glass beaker having an internal volume of 1 L was placed in this ice bath, and 258.3 g of ion-exchanged water was added. The ice bath was installed on a magnetic stirrer, a stirrer tip was added into the beaker, and stirring was carried out.
27.6 g of a 48% potassium hydroxide solution (Kanto Chemical Co., Inc.) was added in small portions into the beaker to produce a potassium hydroxide aqueous solution.
A round-bottomed cylindrical separable flask with an inner diameter of 11 cm and an internal volume of 2 L equipped with a reflux cooling device, a thermometer, and a stirrer (a stirrer blade having four inclined paddle blades having a blade diameter of 5 cm) was prepared. 100 g of an ethylene-acrylic acid copolymer (SK globalchemical.: primacol 5980i) was added to this flask. Thereafter, the total amount of the above-mentioned potassium hydroxide aqueous solution was added. Thereafter, the beaker used for producing the potassium hydroxide aqueous solution was washed with 50.0 g of ion-exchanged water, and the washed water was added to the separable flask to obtain a reaction solution.

While stirring the reaction solution at a rotation speed of 500 rpm of the stirrer, the reaction solution was immersed in an oil bath at 103°C, and the internal temperature was raised to 95°C. Thereafter, the reaction solution was maintained for 4 hours while appropriately adjusting the temperature of the oil bath so that the internal temperature was 95°C to 97°C.

Thereafter, the separable flask was taken out from the oil bath, and left to cool at room temperature until the internal temperature reached 35°C. The internal temperature was confirmed as being 35°C or lower, the reaction solution was filtered through a nylon mesh having an opening of 108 μm to obtain 390.3 g of an emulsion of ethylene-potassium acrylate copolymers serving as the compound A as a filtrate.

<Coating Step>

200 g of the above-mentioned emulsion containing the compound A, and 5.0 g of polyethylene glycol (Tokyo Chemical Industry Co., Ltd., PEG 6000) serving as the compound B were diluted with 295.0 g of ion-exchanged water, to prepare a coating material I. 50.0 g of coated resin particles was obtained in the same manner as in Example 1, except that the coating material I was used instead of the coating material A, and a set temperature of the hot-air dryer was changed to 100°C.

[Example 10]

333.1 g of an emulsion of an ethylene-sodium methacrylate copolymer serving as the compound A was obtained in the same manner as in Example 9, except that the potassium hydroxide aqueous solution was changed to a sodium hydroxide aqueous solution prepared with 7.9 g of sodium hydroxide (FUJIFILM Wako Pure Chemical Corporation) and 259.5 g of ion-exchanged water, and the ethylene-acrylic acid copolymer was changed to 100 g of an ethylene-methacrylic acid copolymer (Dow-Mitsui Polychemicals Co., Ltd.: NUCREL 2050H).

200 g of the above-mentioned emulsion containing the compound A, and 5.0 g of polyethylene glycol (Tokyo Chemical Industry Co., Ltd., PEG 6000) serving as the compound B were diluted with 295.0 g of ion-exchanged water, to prepare a coating material J. 50.0 g of coated resin particles was obtained in the same manner as in Example 1, except that the coating material J was used instead of the coating material A, and a set temperature of the hot-air dryer was changed to 100°C.

[Example 11]

214.5 g of a 23.3% aqueous dispersion emulsion of an acrylic acid-vinyl chloride copolymer (Nissin Chemical co., ltd., VINYBLAN 715) serving as the compound A, and 5.0 g of a polyethylene glycol (Tokyo Chemical Industry Co., Ltd., PEG 6,000) serving as the compound B were diluted with 280.5 g of ion-exchanged water, to prepare a coating material K. 507.9 g of coated resin particles was obtained in the same manner as in Example 1, except that the coating material K was used instead of the coating material A, and heating with the hot-air dryer was not carried out.

[Example 12]

25.0 g of polyvinyl chloride (FUJIFILM Wako Pure Chemical Corporation) serving as the compound A, 2.5 g of polyethylene glycol (Tokyo Chemical Industry Co., Ltd., PEG 6000) serving as the compound B, and 472.5 g of tetrahydrofuran as a solvent were mixed to prepare a coating material L. 506.3 g of coated resin particles was obtained in the same manner as in Example 1, except that the coating material L was used instead of the coating material A, the blowing temperature of the fluid bed granulator during the coating was set to 40°C, and heating with the hot-air dryer was not carried out.

[Example 13]

50.0 g of polystyrene (FUJIFILM Wako Pure Chemical Corporation) serving as the compound A, 5.0 g of polyethylene glycol (Tokyo Chemical Industry Co., Ltd., PEG 6000) serving as the compound B, and 945.0 g of tetrahydrofuran as a solvent were mixed to prepare a coating material M. 509.3 g of coated resin particles was obtained in the same manner as in Example 1, except that the coating material M was used instead of the coating material A, the blowing temperature of the fluid bed granulator during the coating was set to 40°C, and heating with the hot-air dryer was not carried out.

[Example 14]

200.0 g of a 25% aqueous dispersion emulsion of an ethylene-sodium acrylate copolymer (SUMITOMO SEIKA CHEMICALS CO., LTD., ZAIKTHENE N) serving as the compound A, and 5.0 g of sodium chloride (NACALAI TESQUE, INC.) serving as the compound B were diluted with 295.0 g of ion-exchanged water, to prepare a coating material N. 50.0 g of coated resin particles was obtained in the same manner as in Example 1, except that the coating material N was used instead of the coating material A.

[Example 15]

200.0 g of a 25% aqueous dispersion emulsion of an ethylene-sodium acrylate copolymer (SUMITOMO SEIKA CHEMICALS CO., LTD., ZAIKTHENE N) serving as the compound A, and 5.0 g of D-fructose (NACALAI TESQUE, INC.) serving as the compound B were diluted with 295.0 g of ion-exchanged water, to prepare a coating material O. 50.0 g of coated resin particles was obtained in the same manner as in Example 1, except that the coating material O was used instead of the coating material A.

[Comparative Example 2]

200.0 g of a 25% aqueous dispersion emulsion of an ethylene-sodium acrylate copolymer (SUMITOMO SEIKA CHEMICALS CO., LTD., ZAIKTHENE N) serving as the compound A was diluted with 300.0 g of ion-exchanged water, to prepare a coating material P. 50.0 g of coated resin particles was obtained in the same manner as in Example 1, except that the coating material P was used instead of the coating material A.

[Comparative Example 3]

25.0 g of polyvinyl chloride (FUJIFILM Wako Pure Chemical Corporation) serving as the compound A and 475.0 g of tetrahydrofuran as a solvent were added and mixed to prepare a coating material Q. 504.6 g of coated resin particles was obtained in the same manner as in Example 1, except that the coating material Q was used instead of the coating material A, the blowing temperature of the fluid bed granulator during the coating was set to 40°C, and heating with the hot-air dryer was not carried out.

[Comparative Example 4]

50.0 g of polystyrene (FUJIFILM Wako Pure Chemical Corporation) serving as the compound A and 950.0 g of tetrahydrofuran as a solvent were mixed to prepare a coating material R. 506.6 g of coated resin particles was obtained in the same manner as in Example 1, except that the coating material R was used instead of the coating material A, the blowing temperature of the fluid bed granulator during the coating was set to 40°C, and heating with the hot-air dryer was not carried out.

[Comparative Example 5]

50.0 g of polyethylene glycol (Tokyo Chemical Industry Co., Ltd., PEG 6000) serving as the compound B was diluted with 450.0 g of ion-exchanged water, to prepare a coating material S. 505.5 g of coated resin particles was obtained in the same manner as in Example 1, except that the coating material S was used instead of the coating material A, and heating with the hot-air dryer was not carried out.

[Example 16]

200.0 g of a 25% aqueous dispersion emulsion of an ethylene-sodium acrylate copolymer (SUMITOMO SEIKA CHEMICALS CO., LTD., ZAIKTHENE N) serving as the compound A, and 12.5 g of a polyethylene glycol (Tokyo Chemical Industry Co., Ltd., PEG 6,000) serving as the compound B were diluted with 287.5 g of ion-exchanged water, to prepare a coating material T. 50.0 g of coated resin particles was obtained in the same manner as in Example 1, except that the coating material T was used instead of the coating material A.

[Example 17]

Water and ice were added to a plastic tray having a length of 27 cm, a width of 38 cm, and a depth of 7 cm to produce an ice bath at 3°C. A glass beaker having an internal volume of 1 L was placed in this ice bath, and 261.31 g of ion-exchanged water was added. The ice bath was installed on a magnetic stirrer, a stirrer tip was added into the beaker, and stirring was carried out.

9.44 g of sodium hydroxide (granular) (NACALAI TESQUE, INC.) was added in small portions into the beaker to produce a sodium hydroxide aqueous solution.

A round-bottomed cylindrical separable flask with an inner diameter of 11 cm and an internal volume of 2 L equipped with a reflux cooling device, a thermometer, and a stirrer (a stirrer blade having four inclined paddle blades having a blade diameter of 5 cm) was prepared. 100 g of an ethylene-acrylic acid copolymer (SK globalchemical.: primacol 5980i) was added to this flask. Thereafter, the total amount of the above-mentioned sodium hydroxide aqueous solution was added. Thereafter, the beaker used for producing the sodium hydroxide aqueous solution was washed with 50.0 g of ion-exchanged water, and the washed water was added to the separable flask to obtain a reaction solution.

While stirring the reaction solution at a rotation speed of 500 rpm of the stirrer, the reaction solution was immersed in an oil bath at 103°C, and the internal temperature was raised to 95°C. Thereafter, the reaction solution was maintained for 4 hours while appropriately adjusting the temperature of the oil bath so that the internal temperature was 95°C to 97°C.

Thereafter, the separable flask was taken out from the oil bath, and left to cool at room temperature until the internal temperature reached 35°C. The internal temperature was confirmed as being 35°C or lower, the reaction solution was filtered through a nylon mesh having an opening of 108 μm to obtain a 25% aqueous dispersion emulsion of an ethylene-sodium acrylate copolymer having a degree of neutralization of 85% as a filtrate.

200.0 g of a 25% aqueous dispersion emulsion of an ethylene-sodium acrylate copolymer having a degree of neutralization of 85%, produced as the compound A in the above, and 5.0 g of a polyethylene glycol serving as the compound B were diluted with 295.0 g of ion-exchanged water in a polybeaker having an internal volume of 1 L, to prepare a coating material U. 50.0 g of coated resin particles was obtained in the same manner as in Example 1, except that the coating material U was used instead of the coating material A, and heating was carried out with a hot-air dryer set at 100°C.

[Example 18]

In the emulsion production step, an emulsion was produced in the same manner as in Example 9, except that a glass beaker having an internal volume of 1 L was placed in an ice bath, and 897.41 g of ion-exchanged water was added, 10.55 g of sodium hydroxide (granular) was added in small portions into the beaker to produce a sodium hydroxide aqueous solution, thereby obtaining a 10% aqueous dispersion emulsion of an ethylene-sodium acrylate copolymer having a degree of neutralization of 95%.

Thereafter, in the coating step, 500.0 g of a 10% aqueous dispersion emulsion of an ethylene-sodium acrylate copolymer having a degree of neutralization of 95%, produced as the compound A in the above, and 5.0 g of a polyethylene glycol serving as the compound B were mixed with each other in a polybeaker having an internal volume of 1 L, to prepare a coating material V 50.0 g of coated resin particles was obtained in the same manner as in Example 1, except that the coating material V was used instead of the coating material A, and heating was carried out with a hot-air dryer set at 100°C.

[Example 19]

In a step of obtaining the water-absorbent resin particles (1) in the non-coated state, the water-absorbent resin particles (3) in the non-coated state were produced in the same manner as in Comparative Example 1, except that 234 g of water was extracted to the outside of the system while refluxing n-heptane by azeotropic distillation of n-heptane and water, and the coating step was carried out in the same manner as in Example 5 to obtain 50.0 g of coated resin particles. The water retention capacity of the water-absorbent resin particles (3) was 31 g/g.

[Example 20]

In the step of obtaining the water-absorbent resin particles (1) in the non-coated state, the water-absorbent resin particles (4) in the non-coated state were produced in the same manner as in Comparative Example 1, except that 0.00534 g (0.0306 mmol) of ethylene glycol diglycidyl ether (internal crosslinking agent) was added when the first stage monomer aqueous solution was prepared, and the coating step was carried out in the same manner as in Example 5 to obtain 50.0 g of coated resin particles. The water retention capacity of the water-absorbent resin particles (4) was 48 g/g.

2. Evaluation of Absorbent Article

The permeation rate and re-wet were evaluated for absorbers produced using particles for evaluation of Examples 1 to 14 and Comparative Examples 1 to 5.

(Preparation of Resin Particles Used for Absorber)

Mixed particles in a total of 12 g of 9.6 g of the water-absorbent resin particles (2) produced in Comparative Example 1 and 2.4 g of the coated resin particles produced in Examples 1 to 14 and Comparative Examples 2 to 5 were used as absorbers. In addition, only in the case of the absorber of Comparative Example 1, 12 g of the water-absorbent resin particles (2) produced in Comparative Example 1 was used alone.

(Production of Absorber)

12 g of the above-mentioned mixed resin particles for the evaluation and 8.0 g of crushed pulp (manufactured by Rayonier Inc., trade name: Rayfloc) were uniformly mixed by air papermaking to produce an absorber core having a size of 40 cm × 12 cm. Subsequently, an absorber (particle content: 60% by mass) was produced by applying a load of 141 kPa to the entire absorber core for 30 seconds and pressing the absorber core in a state in which the upper and lower parts of the absorber core sandwiched between two sheets of tissue paper (basis weight: 16 g/m$^2$) having the same size as the absorber core.

(Production of Absorbent Article)

An air through type porous liquid permeable sheet (basis weight: 22 g/m$^2$) made of polyethylene having the same size as the absorber was disposed on the upper surface of the above-mentioned absorber, a liquid impermeable sheet (basis weight: 22 g/m$^2$) made of polyethylene having the same size as the absorber was disposed on the lower surface of the absorber to sandwich the absorber, thereby obtaining an absorbent article.

(Preparation of Test Solution)

100.0 g of sodium chloride, 3.0 g of calcium chloride dihydrate, 6.0 g of magnesium chloride hexahydrate, and a mixture of 1.0 g of Triton X-100 (polyoxyethylene(10) octylphenyl ether, manufactured by FUJIFILM Wako Pure Chemical Corporation) and 99.0 g of ion-exchanged water, and 9866.0 g of ion-exchanged water were put in a container having an internal volume of 10 L, and each component was then completely dissolved. Subsequently, the mixed solution was colored with a small amount of Blue No. 1 to prepare a test solution.

(Evaluation of Permeation Rate)

The absorbent article was placed on a horizontal table. A liquid injection cylinder having an opening with an inner diameter of 3 cm was placed on the center of the absorbent article, and 80 mL of the above-mentioned test solution was injected into the cylinder at one time to measure the absorption rate. The same operation was performed a total of five times at intervals of 30 minutes, and a total of the five absorption rates was obtained as a permeation rate [seconds].

(Evaluation of re-wet)

After injecting the test solution five times as mentioned above, the absorbent article was allowed to maintain its state as it was. After 1 hour from the completion of the injection of the test solution, a 10 cm square filter paper whose mass (about 75 g) had been preliminarily measured was placed at around the test solution injection position on the absorbent article. Then, a weight (bottom surface: 10 cm × 10 cm, mass: 5.0 kg (about 0.7 psi)) was placed on the filter paper, loading was then applied for 5 minutes, the mass of the filter paper was measured, and the increase amount of the mass of the filter paper was obtained as the re-wet amount[g].

(Evaluation of Measurement of Dissolved Content)

Compound A (Emulsion)

100 g of the compound A was put into a Teflon (trade name)-coating tray having a bottom dimension of 250 × 185 (mm), and covered by aluminum foil as a lid. The aluminum foil was perforated, and heating was carried out with a hot-air dryer (ADVANTEC, FV-320) at 80°C to form a film.
The obtained polymer film was cut with scissors into a 5 mm square to obtain a coating material. The obtained coating material was added to a similar Teflon (trade name)-coating tray, and covered by aluminum foil as a lid. The aluminum foil was perforated, and heating was carried out with a hot-air dryer (ADVANTEC, FV-320) at 80°C for 2 hours to be completely dried.

100 g of distilled water put into a 200 mL beaker is adjusted to 25°C and stirred at 600 rpm. The completely dried coating material is classified, and 5 g of the coating material remaining on a sieve having an opening of 75 $\mu$m is put into the beaker. The mixture is stirred for 1 hour, and suction-filtration is then carried out by using a 34 $\mu$m stainless steel wire mesh. 60 g of a filtrate obtained by suction filtration is weighed in a pre-weighed 100 mL beaker and dried with a hot-air dryer (TOYO ROSHI KAISHA, LTD., FV-320) at 140°C for 15 hours, and a mass (Wa) of a solid content of the filtrate is measured. A dissolved content is calculated by the following equation.

$$\text{Amount of precipitation (Wb) in case of 100 g of filtrate (g)} =$$

$$(\text{Wa}/60) \times 100$$

$$\text{Dissolved content (\% by mass)} = (\text{Wb}/5) \times 100$$

Compound A (Solution)

10 g of the compound A and 200 g of tetrahydrofuran were mixed to prepare a solution, and the solution was dried with a hot-air dryer at 40°C to form a film. Thereafter, a dissolved content was measured in the same manner as those mentioned in Compound A (Emulsion).

Compound B

The compound B was classified, and a dissolved content was measured in the same manner as those mentioned in Compound A (Emulsion) by using 5 g of the compound B remaining on a sieve having an opening of 75 $\mu$m (where, the film formation and complete drying were not performed).

(Analysis by Raman Spectrometric Method)

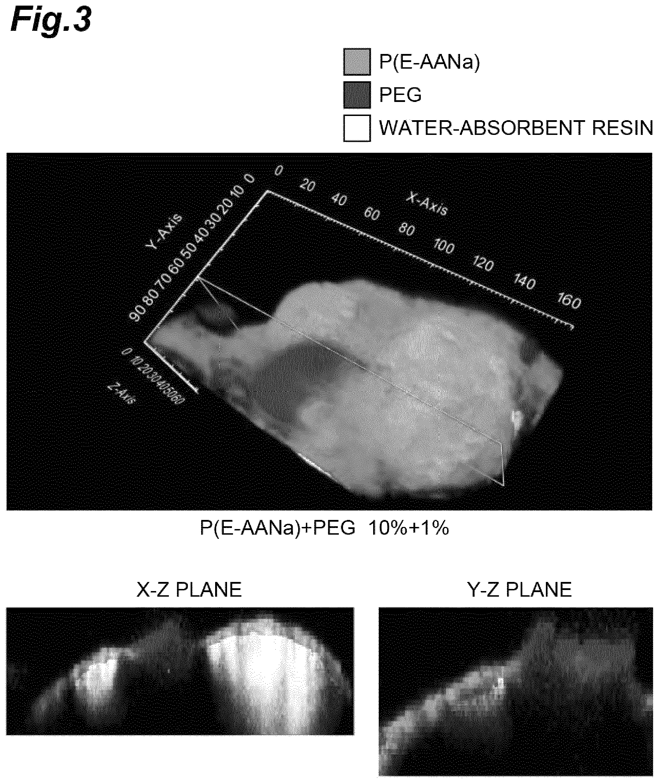

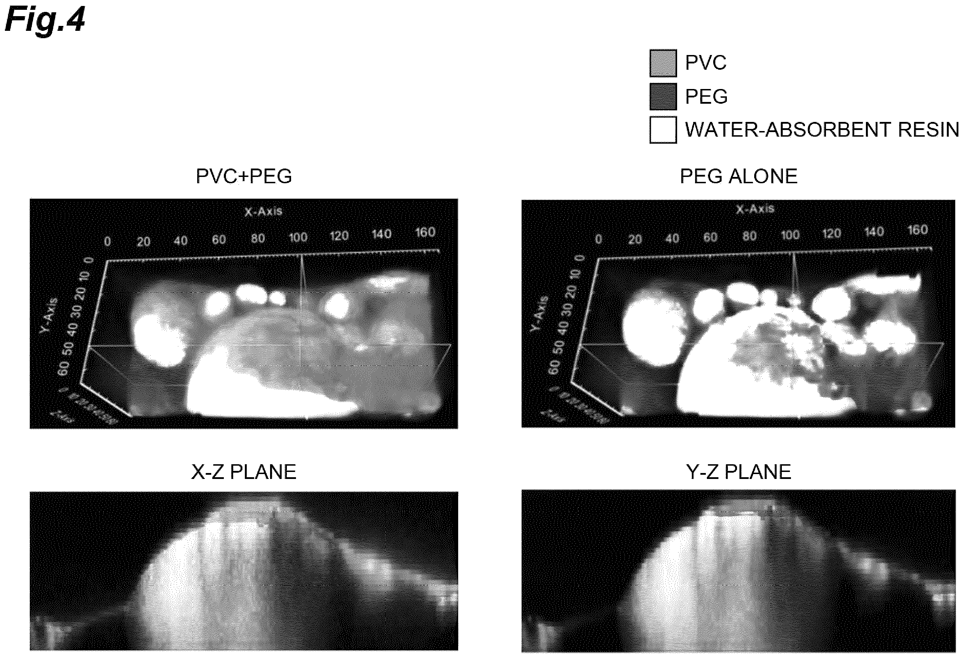

Fig. 3 is a photograph showing an analysis result of the coated resin particle of Example 5 by Raman spectrometric method. Fig. 4 is a photograph showing an analysis result of the coated resin particle of Example 12 by Raman spectrometric method. As shown in Figs. 3 and 4, it was confirmed that the coating layer was formed on at least a part of the surface of the water-absorbent resin particle. As shown in Fig. 3, the coated resin particle of Example 5 had the sea-island structure composed of an sea part containing P(E-AANa) and island part containing PEG. In addition, at least a part of the island part containing PEG constituted the entire thickness of the coating layer.

Measurement by the Raman spectrometric method was performed under conditions shown below.

Instruments used: RAMAN touch (manufactured by Nanophoton Corporation)
Excitation wavelength: 532 nm
Measurement spectrum range: 10 to 4000 cm$^{-1}$
Imaging: line lighting

(Measurement of Water Absorption Inflection Point)

0.200 g of particles for evaluation was precisely weighed and arranged in a layered shape on the bottom of an acrylic cylinder having an inner diameter of 2.0 cm and a depth of 8.0 cm to form a particle layer with a flat upper surface, and a height H0 of the particle layer was then measured. Thereafter, 20 g of physiological saline at 25°C was poured from the upper part of the acrylic cylinder. The measurement was initiated from the time when the total amount of the physiological saline was poured, a height Hn of the particle layer was read at the intervals of 30 seconds (0.5 minutes), the amount of variation in the height per 30 seconds was calculated by the following equation, and a time (minute) of H(n + 1) in a case of the largest variation amount was defined as a time (water absorption inflection point) required for the water absorption rate to increase rapidly. In a case where the upper surface of the particle layer during the water absorption was not flat, a height of the highest portion was set to Hn or H(n + 1).

$$\text{Variation amount (cm)} = \text{H (n + 1)} - \text{Hn}$$

In the above equation, n represents the number of measurements of the height of the particle layer after injecting the physiological saline.

(Evaluation Results)

In Tables 1 and 2, "P(E-AANa)" represents an ethylene-sodium acrylate copolymer, "P(E-AAK)" represents an ethylene-potassium acrylate copolymer, "P(E-MAANa)" represents an ethylene-sodium methacrylate copolymer, "(AA-VC)" represents an acrylic acid-vinyl chloride copolymer, "PVC" represents polyvinyl chloride, and "PS" represents polystyrene.

[Table 1]

| | Water-absorbent resin particles used | Coated resin particles | | | | | | | | | | Water-absorbent characteristics | Evaluation of absorber | | |
|---|---|---|---|---|---|---|---|---|---|---|---|---|---|---|---|
| | | Coating material | | | | | | | | | | | | | |
| | | Type | | | | Adding amount vs water-absorbent resin particles (%) | | Dissolved content (% by mass) | | | Water retention capacity (g/g) | Permeation rate (second) | Re-wet (g) | Water absorption inflection point (minute) |
| | | A | | | B | A | B | A | B | B-A | | | 260 seconds or less | 70 g or less | |
| | | Composition | Degree of neutralization | Form | | | | | | | | | | | |
| Example 1 | (1) | P(E-AANa) | 75 | Emulsion | PEG | 0.5 | 0.05 | 0 | 82 | 82 | 39 | 259 | 63 | - |
| Example 2 | (1) | P(E-AANa) | 75 | Emulsion | PEG | 2 | 0.2 | 0 | 82 | 82 | 39 | 233 | 65 | 1.5 |
| Example 3 | (1) | P(E-AANa) | 75 | Emulsion | PEG | 30 | 3 | 0 | 82 | 82 | 30 | 212 | 65 | 4.0 |
| Example 4 | (1) | P(E-AANa) | 75 | Emulsion | PEG | 10 | 0.1 | 0 | 82 | 82 | 38 | 231 | 65 | - |
| Example 5 | (1) | P(E-AANa) | 75 | Emulsion | PEG | 10 | 1 | 0 | 82 | 82 | 38 | 232 | 66 | 3.5 |
| Example 16 | (1) | P(E-AANa) | 75 | Emulsion | PEG | 10 | 2.5 | 0 | 82 | 82 | 38 | 236 | 66 | 3.0 |
| Example 6 | (1) | P(E-AANa) | 75 | Emulsion | PEG | 1 | 5 | 0 | 82 | 82 | 39 | 238 | 68 | - |
| Example 7 | (1) | P(E-AANa) | 75 | Emulsion | PEG | 1 | 10 | 0 | 82 | 82 | 39 | 256 | 65 | - |
| Example 19 | (3) | P(E-AANa) | 75 | Emulsion | PEG | 10 | 1 | 0 | 82 | 82 | 28 | 210 | 68 | 2.5 |
| Example 20 | (4) | P(E-AANa) | 75 | Emulsion | PEG | 10 | 1 | 0 | 82 | 82 | 46 | 259 | 63 | 2.5 |
| Example 8 | Particles collected from diaper | P(E-AANa) | 75 | Emulsion | PEG | 10 | 1 | 0 | 82 | 82 | 28 | 206 | 60 | - |
| Example 17 | (1) | P(E-AANa) | 85 | Emulsion | PEG | 10 | 1 | 0 | 82 | 82 | 38 | 200 | 65 | 5.0 |
| Example 18 | (1) | P(E-AANa) | 95 | Emulsion | PEG | 10 | 1 | 0 | 82 | 82 | 38 | 195 | 66 | 5.5 |

[Table 2]

| | Water-absorbent resin particles used | Coated resin particles | | | | | | | | | | Water-absorbent characteristics | Evaluation of absorber | | Water absorption inflection point (minute) |
|---|---|---|---|---|---|---|---|---|---|---|---|---|---|---|---|
| | | Coating material | | | | Adding amount vs water-absorbent resin particles (%) | | Dissolved content (% by mass) | | | | | Permeation rate (second) | Re-wet (g) | |
| | | Type | | | | | | | | | | Water retention capacity (g/g) | 260 seconds or less | 70 g or less | |
| | | A | | | B | A | B | A | B | B-A | | | | | |
| | | Composition | Degree of neutralization | Form | | | | | | | | | | | |
| Example 9 | (1) | P(E-AAK) | 75 | Emulsion | PEG | 10 | 1 | 0 | 82 | 82 | | 38 | 208 | 66 | 5.0 |
| Example 10 | (1) | P(E-MAANa) | 75 | Emulsion | PEG | 10 | 1 | 0 | 82 | 82 | | 36 | 221 | 68 | 6.0 |
| Example 11 | (1) | P(AA-VC) | - | Emulsion | PEG | 10 | 1 | 0 | 82 | 82 | | 36 | 237 | 65 | - |
| Example 12 | (1) | PVC | - | Solution | PEG | 5 | 0.5 | 0 | 82 | 82 | | 29 | 241 | 69 | - |
| Example 13 | (1) | PS | - | Solution | PEG | 10 | 1 | 0 | 82 | 82 | | 38 | 250 | 67 | - |
| Example 14 | (1) | P(E-AANa) | 75 | Emulsion | NaCl | 10 | 1 | 0 | 54 | 54 | | 37 | 222 | 65 | - |
| Example 15 | (1) | P(E-AANa) | 75 | Emulsion | Fructose | 10 | 1 | 0 | 85 | 85 | | 36 | 223 | 66 | - |
| Comparative Example 1 | (2) | Non-coated | | | | - | - | - | - | - | | 40 | 263 | 62 | 0.5 |
| Comparative Example 2 | (1) | P(E-AANa) | 75 | Emulsion | - | 10 | 0 | 0 | - | - | | 33 | 230 | 72 | - |
| Comparative Example 3 | (1) | PVC | - | Solution | - | 5 | 0 | 0 | - | - | | 29 | 235 | 74 | - |
| Comparative Example 4 | (1) | PS | - | Solution | - | 10 | 0 | 0 | - | - | | 32 | 244 | 71 | - |
| Comparative Example 5 | (1) | - | - | - | PEG | - | 10 | - | 82 | - | | 39 | 279 | 63 | - |

21

It was shown that in the case where the coated resin particles of the examples were used for the absorbent article, the permeation rate was improved while suppressing re-wet after liquid absorption.

Based on a comparison of the result of Example 2 and the result of Example 3, it can be seen that even in a case where the sea part and the island part are present in the same ratio, the water absorption inflection point extends as the use amount of the coating material is larger.

Based on a comparison of the result of Example 5 and the result of Example 16, it can be seen that the water absorption inflection point is shortened as the ratio of the island part to the sea part is larger.

Based on a comparison of the result of Example 17 and the result of Example 18, it can be seen that the water absorption inflection point extends as the degree of neutralization of the sea part is higher. It is presumed that the water absorption inflection point extends according to an increase in the degree of neutralization of the sea part because an emulsion is made smaller particles to enable a dense coating layer as the degree of neutralization is higher.

Based on a comparison of the result of Example 5, the result of Example 9, and the result of Example 10, it can be seen that the water absorption inflection point can be controlled even though the composition of the sea part (emulsion resin) is changed. In particular, as can be seen from a comparison of the result of Example 5 and the result of Example 9, the water absorption inflection point can also be changed by the change of the counterion.

From the above-mentioned results, it is confirmed that in a case of (1) the type and/or use amount of the coating material, (2) the ratio between the sea part and the island part, and (3) using the polymer containing a carboxy group such as EAA for forming the sea part, the water absorption inflection point can be controlled by the degree of neutralization of the sea part.

**Reference Signs List**

1, 10a: Coated resin particle,
1a: Water-absorbent resin particle,
1b: Coating layer,
10: Absorber,
10b: Fiber layer,
20a, 20b: Core wrap,
30: Liquid permeable sheet,
40: Liquid impermeable sheet,
100: Absorbent article.

**Claims**

1. A coated resin particle comprising:

   a water-absorbent resin particle; and
   a coating layer covering at least a part of a surface of the water-absorbent resin particle,
   wherein the coating layer comprises a compound A and a compound B more water-soluble than the compound A.

2. The coated resin particle according to claim 1,

   wherein a dissolved content of the compound A in 100 g of water at 25°C is 1% by mass or less, and
   a dissolved content of the compound B in 100 g of water at 25°C is higher than the dissolved content of the compound A by 20% by mass or more.

3. The coated resin particle according to claim 1 or 2,
   wherein a content of the compound B is 1 to 85 parts by mass with respect to 100 parts by mass in a total content of the compound A and the compound B.

4. The coated resin particle according to any one of claim 1 to 3,
   wherein the coating layer comprises a sea-island structure composed of a sea part comprising one compound of the compound A and the compound B, and an island part comprising the other compound.

5. The coated resin particle according to claim 4,

wherein the sea part comprises the compound A, and
the island parts comprise the compound B.

6. A water-absorbent resin composition comprising:

the coated resin particle according to any one of claims 1 to 5; and
a resin particle having a water absorption property other than the coated resin particle.

7. A method for producing a coated resin particle, the method comprising:
a step of coating at least a part of the water-absorbent resin particle with a coating material to form a coating layer on at least a part of a surface of the water-absorbent resin particle,
wherein the coating material comprises a compound A in a solution state or an emulsion state, and a compound B more water-soluble than the compound A.

8. The method for producing a coated resin particle according to claim 7,
wherein a content of the compound B is 1 to 500 parts by mass with respect to 100 parts by mass of a content of the compound A.

Fig.1

*Fig.2*

# Fig.3

■ P(E-AANa)
■ PEG
□ WATER-ABSORBENT RESIN

P(E-AANa)+PEG 10%+1%

X-Z PLANE

Y-Z PLANE

## *Fig.4*

PVC
PEG
WATER-ABSORBENT RESIN

PVC+PEG

PEG ALONE

X-Z PLANE

Y-Z PLANE

## INTERNATIONAL SEARCH REPORT

| International application No. |
|---|
| **PCT/JP2022/017872** |

**A.    CLASSIFICATION OF SUBJECT MATTER**

*C08J 3/12*(2006.01)i; *C08L 101/12*(2006.01)i; *A61F 13/53*(2006.01)i; *B01J 20/26*(2006.01)i; *B01J 20/28*(2006.01)i; *B01J 20/32*(2006.01)i; *A61L 15/22*(2006.01)i; *A61L 15/24*(2006.01)i; *A61L 15/42*(2006.01)i; *A61L 15/60*(2006.01)i
FI:    C08J3/12 Z CER; B01J20/28 A; B01J20/28 Z; B01J20/32; A61L15/24 100; A61L15/42 100; A61L15/60 100; A61L15/22 200; A61L15/60 200; A61F13/53 300; C08J3/12 CEZ; C08L101/12; B01J20/26 D

According to International Patent Classification (IPC) or to both national classification and IPC

**B.    FIELDS SEARCHED**

Minimum documentation searched (classification system followed by classification symbols)

C08J3/12; C08L101/12; A61F13/53; B01J20/26; B01J20/28; B01J20/32; A61L15/22; A61L15/24; A61L15/42; A61L15/60

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | JP 02-242858 A (SANYO CHEMICAL IND LTD) 27 September 1990 (1990-09-27) claims, p. 2, lower right column, line 7 to p. 3, lower left column, line 20, p. 4, upper right column, line 9 to p. 6, upper left column, line 11, examples | 1-5, 7, 8 |
| Y | | 6 |
| X | JP 2002-501088 A (BASF AKTIENGESELLSCHAFT) 15 January 2002 (2002-01-15) claims, paragraphs [0007], [0008], [0035]-[0047], examples, in particular, example 2 | 1-5, 7, 8 |
| Y | | 6 |
| X | JP 64-033149 A (NIPPON SYNTHETIC CHEM IND CO LTD) 03 February 1989 (1989-02-03) claims, p. 3, upper left column, line 6 to p. 6, lower left column, line 15, examples | 1-5, 7, 8 |
| Y | | 6 |
| Y | JP 2007-244442 A (KAO CORP) 27 September 2007 (2007-09-27) claims, examples | 6 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **21 June 2022** | **05 July 2022** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

## INTERNATIONAL SEARCH REPORT

| | International application No. |
|---|---|
| | **PCT/JP2022/017872** |

**C. DOCUMENTS CONSIDERED TO BE RELEVANT**

| Category* | Citation of document, with indication, where appropriate, of the relevant passages | Relevant to claim No. |
|---|---|---|
| A | JP 2011-511136 A (EVONIK STOCKHAUSEN, INC) 07 April 2011 (2011-04-07) claims, examples, entire text | 1-8 |
| A | JP 56-159232 A (KURARAY CO) 08 December 1981 (1981-12-08) claims, examples, entire text | 1-8 |
| A | WO 2014/119553 A1 (NIPPON SHOKUBAI CO., LTD.) 07 August 2014 (2014-08-07) claims, examples, entire text | 1-8 |
| P, X | WO 2021/117786 A1 (SUMITOMO SEIKA CHEMICALS) 17 June 2021 (2021-06-17) claims, paragraphs [0071]-[0100], examples, in particular, example 5 | 1-8 |
| P, X | WO 2021/117782 A1 (SUMITOMO SEIKA CHEMICALS) 17 June 2021 (2021-06-17) claims, paragraphs [0080]-[0126], examples, in particular, example 7 | 1-8 |

Form PCT/ISA/210 (second sheet) (January 2015)

## INTERNATIONAL SEARCH REPORT
### Information on patent family members

International application No.

**PCT/JP2022/017872**

| Patent document cited in search report | | | Publication date (day/month/year) | Patent family member(s) | | | Publication date (day/month/year) |
|---|---|---|---|---|---|---|---|
| JP | 02-242858 | A | 27 September 1990 | (Family: none) | | | |
| JP | 2002-501088 | A | 15 January 2002 | WO | 1999/037395 | A1 | |
| | | | | EP | 1049535 | A1 | |
| | | | | DE | 19801933 | A1 | |
| | | | | CA | 2316342 | A | |
| JP | 64-033149 | A | 03 February 1989 | (Family: none) | | | |
| JP | 2007-244442 | A | 27 September 2007 | (Family: none) | | | |
| JP | 2011-511136 | A | 07 April 2011 | US | 2009/0191408 | A1 | |
| | | | | claims, examples | | | |
| | | | | US | 2013/0096000 | A1 | |
| | | | | WO | 2009/097420 | A2 | |
| | | | | EP | 2234651 | A2 | |
| | | | | EP | 2653173 | A2 | |
| | | | | CN | 101932343 | A | |
| | | | | KR | 10-2010-0130590 | A | |
| | | | | TW | 200940629 | A | |
| | | | | TW | 201425420 | A | |
| | | | | KR | 10-2016-0056326 | A | |
| JP | 56-159232 | A | 08 December 1981 | (Family: none) | | | |
| WO | 2014/119553 | A1 | 07 August 2014 | US | 2015/0360204 | A1 | |
| | | | | claims, examples | | | |
| | | | | EP | 2952537 | A1 | |
| | | | | KR | 10-2015-0113042 | A | |
| WO | 2021/117786 | A1 | 17 June 2021 | WO | 2021/117780 | A1 | |
| | | | | WO | 2021/117781 | A1 | |
| | | | | WO | 2021/117782 | A1 | |
| | | | | WO | 2021/117783 | A1 | |
| | | | | WO | 2021/117784 | A1 | |
| | | | | WO | 2021/117785 | A1 | |
| WO | 2021/117782 | A1 | 17 June 2021 | WO | 2021/117780 | A1 | |
| | | | | WO | 2021/117781 | A1 | |
| | | | | WO | 2021/117783 | A1 | |
| | | | | WO | 2021/117784 | A1 | |
| | | | | WO | 2021/117785 | A1 | |
| | | | | WO | 2021/117786 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014046020 A